(19) Europäisches Patentamt

European Patent Office

Office européen des brevets

(11) Publication number: **0 330 185 B1**

(12) **EUROPEAN PATENT SPECIFICATION**

(45) Date of publication of patent specification: 29.09.93 (51) Int. Cl.⁵: **C12Q 1/68**, G01N 33/543

(21) Application number: **89103103.1**

(22) Date of filing: **22.02.89**

(54) **Method for assaying genetic sequences and kit therefor.**

(30) Priority: **26.02.88 US 161911**

(43) Date of publication of application:
**30.08.89 Bulletin 89/35**

(45) Publication of the grant of the patent:
**29.09.93 Bulletin 93/39**

(84) Designated Contracting States:
**AT BE CH DE ES FR GB GR IT LI LU NL SE**

(56) References cited:
EP-A- 0 238 332      WO-A-86/03227
WO-A-87/03622      US-A- 4 436 826
US-A- 4 454 233      US-A- 4 663 277

(73) Proprietor: **PROFILE DIAGNOSTIC SCIENCES INC.**
**400 Valley Road**
**Warrington Pennsylvania 18976-2590(US)**

(72) Inventor: **Wang, Chia-Gee**
**44 Glenwood Road**
**Millwood, NY 10546(US)**

(74) Representative: **Baillie, Iain Cameron et al**
**c/o Ladas & Parry, Altheimer Eck 2**
**D-80331 München (DE)**

**Description**

This invention relates to a method for assaying genetic sequences and to a kit useful for the assay of such sequences.

The identification of genetic sequences is an important procedure in biological, medical and biotechnology research. Identification of and quantitative information about portions of genetic material, i.e. genetic sequences, is of great interest in a wide variety of applications. Such information, for example, enables identification of cells or organisms, or even of individual higher organisms through "genetic fingerprints". Knowledge about the presence and quantity of specific genetic material is of importance in research, diagnosis and treatment of diseases. There is also great interest in mapping genes by identification of genetic fragments or sequences. A number of methods currently in use for identifying such genetic sequences are typified by the "Southern blotting" procedure which employs a genetic probe to identify related genetic fragments. A genetic probe is a single strand of DNA (or RNA) from a fragment of genetic material normally of known nucleotide sequence. The probe identifies identical or nearly identical complementary genetic sequences by the formation of complementary double helices or duplexes. In the Southern blotting technique, genetic fragments to be identified are first separated according to size in a gel by electrophoresis, the separated fragments are transferred to a nitrocellulose sheet without change of relative position of the fragments and a specific radioactively labelled genetic probe is applied to the sheet. Subsequently, an autoradiograph is obtained which shows the restriction fragments which hybridized with the genetic probe to form radioactive hybrids or duplexes.

EP-A-0 304 845 describes and claims a method of assaying gene expressions which comprises linking mRNA molecules to a solid substrate and contacting the solid substrate with an aqueous suspension of labelled microbeads to which gene probe molecules are linked. The gene probe molecules comprise sequences which hybridize with sequences comprised by and characteristic of the unknown or target mRNA molecules to be assayed, so that the labelled microbeads are linked to the target mRNA molecules on the substrate. By separating the solid substrate from microbeads unlinked to target mRNA molecules and detecting labelled, linked microbeads, the gene expressions are assayed. The disclosed preferred detection method is by X-ray fluorescence of metal element labels.

Cloning, hybridization and radioactive gene probe techniques are described in detail in the text "Molecular Cloning", T. Maniatis et al., Cold Spring Harbor Laboratory, 1982.

Labelled microbeads have been used in a number of analytical methods such as immunoassay methods. Extensive prior art concerning microbeads is described in U.S. Patents 4,454,233 granted June 12, 1984 and 4,436,826 granted March 13, 1984, both granted to the present applicant Chia-Gee Wang. These patents disclose the use of microspheres to couple with an antibody detector, and the use of tagging or labelling reporters which con be detected by a number of methods including X-ray fluorescence. U.S. Patent No. 4,663,277 granted on May 5, 1987 also to the present applicant describes a method for the detection of viruses or proteins using a solid phase amplification technique. The target viruses or proteins in a specimen are contacted with a solid substrate coated with on antibody which forms a complex with the target, and microspheres coated with antibody are then bound to the target complex. The detection sensitivity is amplified by the labelled microspheres. The use of X-ray fluorescence analysis is disclosed, and is shown to enable the simultaneous determination of different kinds of targets.

According to the present invention there is provided a method for assaying genetic sequences, which comprises admixing in an aqueous medium under predetermined assay conditions (1) genetic material which includes a sequence to be assayed (target sequence) and (2) weakly hybridized nucleotide duplexes of (i) a genetic probe for said target sequence, wherein the target sequence comprises a characteristic sense sequence and the genetic probe comprises an antisense sequence complementary to the characteristic sense sequence of said target sequence, and (ii) a reporter molecule linked to a labelled microbead, said genetic probe being capable of hybridizing more strongly with said target sequence than with said reporter molecule under said predetermined assay conditions; whereby target sequences hybridize with genetic probes of said duplexes and, correspondingly, reporter molecules disassociate from said probe-reporter duplexes;
differentiating disassociated reporter molecules from undisassociated probe-reporter duplexes;
and detecting the labels of the microbeads which are linked to the differentiated, disassociated reporter molecules.

Also provided in accordance with the present invention is a kit for assaying genetic sequences in an aqueous medium containing genetic material which includes a genetic target sequence to be assayed, said kit comprising an aqueous medium containing together in said medium or in separate portions of the medium the following components: A. weakly hybridized nucleotide duplexes of (i) a genetic probe for said target sequence and (ii) a reporter molecule linked to a labelled microbead; and B. a plurality of nucleotide-comprising homopolymers capable of selectively hybridizing with the reporter molecules (ii) when said reporter molecules are not hybridized with the genetic probes (i).

In a further embodiment of the present invention there is provided a method for assaying genetic sequences, which comprises admixing in an aqueous medium under predetermined assay conditions (I) genetic material which includes a sequence to be assayed (target sequence) and (II) weakly hybridized nucleotide duplexes of (a) a genetic probe for said target sequence, said genetic probe being linked to a labelled microbead, and (b) a homopolymer molecule, wherein the target sequence comprises a characteristic sense sequence and the genetic probe comprises (1) an antisense sequence complementary to the characteristic sense sequence of said target sequence and (2) a hybridizable sequence adapted to hybridize with said homopolymer, said genetic probe being capable of hybridizing more strongly with said target sequence than with said homopolymer under said predetermined assay conditions; whereby target sequences hybridize with genetic probes of the probe-homopolymer duplexes to form target-probe duplexes and the labelled microbeads carried by the genetic probes of the formed target-probe duplexes are disassociated from said homopolymers; segregating the remaining probe-homopolymer duplexes from target-probe duplexes; and detecting the labels of the labelled microbeads in the remaining probe-homopolymer duplexes to measure the decrease from a count Of labels in the initially provided probe-homopolymer duplexes.

In said further embodiment, there is also provided in accordance with the present invention a kit for assaying genetic sequences in an aqueous medium containing genetic material which includes a genetic target sequence to be assayed, said kit comprising an aqueous medium containing component (A) which comprises weakly hybridized nucleotide duplexes of (a) a genetic probe for said target sequence, said genetic probe being linked to a labelled microbead, and (b) a homopolymer molecule, said genetic probe being capable of hybridizing more strongly with said target sequence than with said homopolymer under predetermined assay conditions.

Prior art techniques such as the Southern blotting procedure have involved a heterogeneous assay in which the material being assayed is bound to a solid and a detector such as a gene probe, applied in a liquid phase, has to bind to the remaining binding sites on the material being analyzed. Since a considerable number of binding sites may be rendered unavailable by binding the material being analyzed to a solid, sensitivity of the assay is lowered. Moreover, the prior techniques involve a series of time-consuming manipulative steps.

There is a need for a method for assaying genetic materials with greater sensitivity or fewer manipulative steps. The present invention provides a homogeneous assay technique in which a profile of genetic sequences being assayed is treated in a liquid phase and can be quickly determined with a minimum of manipulative steps.

FIG. 1 is a schematic illustration of one embodiment of the assay of the present invention.

FIG. 2 is a schematic illustration of another embodiment of the assay of the present invention.

Nucleic acids as genetic material are found in all living cells. The two types of nucleic acid, deoxyribonucleic acid (DNA) and ribonucleic acid (RNA), differ from each other primarily in their ribose sugar unit. Each microorganism contains a unique sequence of nucleotides and can be identified by specific portions of its DNA or RNA, particularly by its abundant ribosomal RNA (rRNA). In higher organisms, besides the unique DNA sequence, each cell of certain tissues contains a set of gene expressions in the form of messenger RNA (mRNA). This unique characteristic provides a powerful means of detecting and identifying the organisms or cells by use of genetic probes.

Genetic probes are single-stranded chains of nucleic acid having a specific sequence ("antisense sequence") that can hybridize specifically, under appropriate assaying conditions, with a "sense sequence" of the target nucleic acid sequences. The target sequence can be DNA, rRNA, or mRNA. DNA is normally double stranded, whereas RNA is normally single stranded. A sequence of a double stranded "duplex" can be denatured into two complementary single stranded sequences at an elevated temperature, with higher melting temperature for longer duplexes.

An assay using genetic probes involves two functions, detecting and reporting. The unique probe sequence detects the sense of the target sequence by hybridizing with it, forming an antisense-sense duplex. Upon hybridization, this antisense-sense duplex must report its presence by reporters, which can be, e.g., tagged enzyme molecules, fluorescent molecules, radioactive atoms, or elements or compounds detectable by X-ray fluorescence. In the present invention, microbeads containing several hundred million tracing atoms are used to form labelled reporters.

By using microbeads to form the reporter, each reporter carries several hundred million tracing atoms of a particular element, and serves as one reporting unit of a mobile solid phase. The large number of tracing atoms in each reporting unit amplifies the reporting signal greatly in contrast to that of using a single radioactive atom or color altering molecule as a label. Such microbeads are particularly convenient in X-ray fluorescent measurements where ten or more reporting channels, each tagged with a different trace element, can report simultaneously, serving the functions of assaying a profile of target genetic sequences simultaneously as well as calibrating the assay internally.

The size of such microbeads, however, remains at typically that of a virus so that while they can form the reporter by covalently linking with nucleotide sequences, they do not form an unduly large assembly which would adversely affect the designated detecting conjugations.

In accordance with the method of the present invention, genetic target sequences, which can be either DNA or RNA single stranded molecules, are assayed in an aqueous suspension or medium. A previously hybridized duplex of a genetic probe for the target sequence and a reporter molecule linked to a labelled microbead is admixed in the medium with genetic material which includes one or more sequences to be assayed (target sequences). The duplex is a weakly hybridized nucleotide duplex and the genetic probe is capable of hybridizing more strongly with the target sequence than with the reporter molecule under predetermined assayed conditions. Under such conditions, the genetic probe preferentially hybridizes with the target sequence so that correspondingly the existing probe-reporter duplex is disassociated, releasing the reporter molecules which are linked to labelled microbeads.

Detection of the labels of the microbeads which are linked to disassociated reporter molecules is a measure of the presence and quantity of target sequences. To detect the labels of microbeads of disassociated reporter molecules, it is necessary to differentiate the disassociated reporter molecules from undisassociated probe-reporter duplexes which themselves contain the labelled microbeads. Differentiation can be done in a number of ways such as by collecting disassociated reporter molecules on a solid substrate and removing the aqueous medium.

The labels of the microbeads may for example be colored compounds, compounds which are fluorescent under ultraviolet illumination, radioactive tracers or metal elements or compounds detectable by X-ray fluorescence. It is preferred that the labels should be metal elements or compounds which are detectable by X-ray fluorescence. Preferably, the labels are relatively heavy elements having an atomic number higher than 22 so that their X-ray fluorescence are not strongly attenuated in the air. Examples of such elements are Ti, Fe, Ni, Cu, Co, Cr and the like.

The preferred detection procedure is X-ray fluorescence analysis. In a typical X-ray fluorescence spectrum, each trace element has its characteristic peak position or energy. The counting rate at that position or channel is proportional to the number of microbeads tagged with the corresponding element. The fluorescence is measured in the presence of air, involving about $10^{-8}$ mole of argon gas near the X-ray detector. The channel of the argon signal does not include the amplifying factor of the microbead labels. Using microbeads, assuming each contributing an amplifying factor of $10^8$, it is implied that signal level of $10^{-16}$ mole for the target molecules can be reached in a few seconds. The assay sensitivity using such a system is, of course, very much higher.

The microbeads used in the present invention preferably are small latex beads such as those supplied by Polyscience, Inc. (Paul Valley Industrial Park, Warrington, PA 18976). A preferred size (diameter) of the microbeads is 0.3 micrometers or less. To improve sensitivity, is preferred that the microbeads should be 0.25 micrometers or smaller, such as 0.1 micrometers or below. The microbeads preferably are not smaller than 0.05 micrometers since below this size the reporting signals are reduced.

The weak hybridizing characteristics of the genetic probe/reporter molecule duplex and the stronger hybridizing of the genetic probe with the target sequence under given assay conditions can result from the respective number of nucleotide base pairs which form the duplex by complementary binding. In an embodiment of the invention, the target sequence comprises a characteristic sense sequence, the genetic probe comprises an antisense sequence complementary to the characteristic sense sequence of the target sequence and the reporter molecule comprises a sense sequence which is shorter than the target sense sequence and is complementary to part of the genetic probe antisense sequence. This can result in the preferential binding of the probe to the target under the assay conditions. Such conditions as temperature or pH are predetermined so that the genetic probe forms a weakly hybridized duplex with the reporter molecule and a strongly hybridized duplex with the target sequence. For this purpose it is preferred that the assay be conducted at a temperature just below the melting temperature of the duplex of the genetic probe and the reporter molecule.

One way of differentiating the disassociated reporter molecules from undisassociated probe-reporter duplexes is to combine the disassociated reporter molecules with another substance, so that distinguishing characteristics can be imparted to the disassociated reporter molecule or so that it can be collected at a position different from the position of undisassociated probe-reporter duplexes. One way of doing this is to provide in the aqueous medium a number of nucleotide polymers which have characteristics that they will selectively hybridize with only the disassociated reporter molecules.

Preferably, the nucleotide polymers which may be provided in the medium are nucleotide-comprising homopolymer molecules.

The reporter molecule comprises a hybridizable sequence adapted to hybridize with the homopolymer. A number of nucleotides which are capable of forming base pairs with the homopolymer may be comprised by that hybridizable sequence. Alternatively, nuleotides capable of forming base pairs with the homopolymer may be comprised by both the hybridizable sequence and the sense sequence of the reporter molecule. The hybridization of the homopolymer and reporter molecule then will depend on the number of nucleotides available for forming base pairs and on the assay conditions. A preferred embodiment of the invention is where the number of base pair-forming nucleotides is sufficient under the predetermined assay conditions for the formation of a reporter-homopolymer duplex between the hybridizable sequence and the homopolymer when the reporter molecule is disassociated from the genetic probe, and is insufficient under the reaction conditions to form the reporter-homopolymer duplex when the reporter molecule is hybridized with the genetic probe due to blocking of some of said base pair-forming nucleotides by the genetic probe. When the base pair-forming nucleotides are comprised by both the hybridizable sequence and the sense sequence of the reporter molecule, it is preferred for the base pair-forming nucleotides of the sense sequence to be located adjacent to the hybridizable sequence of the reporter.

The above homopolymers may be composed of X nucleotides and the base pair-forming nucleotides of the reporter molecule of Y nucleotides, wherein when:

X is dC, Y is G;

X is C, Y is G;

X is dG, Y is C;

X is G, Y is C;

X is dC, Y is dG;

X is C, Y is dG;

X is dG, Y is dC;

X is G, Y is dC.

When the disassociated reporter molecules containing the labelled microbeads are differentiated by hybridizing with homopolymers in the medium, the resulting duplex of homopolymer and reporter molecules may be distinguished from probe-reporter molecules in various ways. One such way is to provide the homopolymers in a fixed location. This may be done by linking the homopolymers to a solid substrate on which the disassociated reporters are collected by formation of reporter-homopolymer duplexes. The solid substrate may be composed of any material to which the homopolymers can be linked, such as glass or a hydrophilic polymeric latex. For example, the substrate could be a container within which the aqueous medium is contained, and the homopolymers could be linked to a specific location of the surface inside the container.

Providing the homopolymers linked at a specific location on such a solid substrate enables an advantageous method of detection to be employed. This detection procedure can be followed when the microbeads are labelled with metal elements or compounds. The labelled microbeads linked to disassociated reporter molecules then may be detected by X-ray fluorescence analysis utilizing a source of X-rays or electrons for irradiating the disassociated reporter molecules collected on the solid substrate and an X-ray fluorescence detector capable of viewing a narrow solid angle. For this purpose, the fixed location of the homopolymers on the solid substrate is selected in relation to the detector and the aqueous medium so that the detector essentially views fluorescent X-rays emitted by the collected disassociated reporter molecules without viewing a significant number of fluorescent X-rays emitted by undisassociated reporter molecules in said aqueous medium. This in effect segregates the disassociated reporter molecules from the undisassociated ones.

An alternative procedure for detection of the labels of undisassociated reporter molecules collected in a fixed location by the formation of the reporter-homopolymer duplexes, is to remove the aqueous medium from the location of the solid substrate, and where necessary, to wash the solid substrate. This results in removing undisassociated probe-reporter duplexes from the location of the solid substrate at which the homopolymer-reporter duplexes are affixed. Then, the labels of the reporter molecules in such location can be detected by any suitable means, since the detection will not be affected by "noise" signals resulting from the presence of the labels of reporter molecules in the undisassociated probe-reporter duplexes.

In the case of use of homopolymer molecules in the aqueous medium, procedures other than affixing the homopolymers to a solid substrate can be followed. For example, the homopolymer molecules may be free-floating, and the resulting homopolymer-reporter duplexes can be distinguished from undisassociated probe-reporter duplexes. One way of doing this is to label the homopolymer molecules. Each homopolymer molecule may be linked to a magnetically labelled microbead. Disassociated, labelled reporter molecules then may be differentiated from undisassociated probe-reporter duplexes by the formed reporter-homopolymer duplexes having magnetic labels. Such magnetically labelled reporter-homopolymer duplexes can be collected by magnetic attraction to a desired location. At the location the labels of microbeads linked to reporter molecules can be detected. Undesired interference from undisassociated probe-reporter duplexes can be avoided by the means described above such as removal of the aqueous medium from the collected duplexes.

The method of the invention also may employ a control assay done simultaneously with the assay of an unknown target sequence. This may be done by also admixing in the aqueous medium as a control a known quantity of a known target sequence and additional weakly hybridized nucleotide duplexes. These additional duplexes are formed from a genetic probe for the known target sequence and a reporter molecule linked to a labelled microbead, with that microbead being labelled with a metal element different from the label of the reporter molecule for the unknown target sequence. The genetic probe for the known target sequence is capable of hybridizing more strongly with the known target sequence than with the reporter molecule to which it is initially hybridized, under predetermined assay conditions. When both the unknown and the known target sequences have been admixed, the detection step simultaneously and separately detects both labels of microbeads linked to reporter molecules disassociated from the genetic probe for the known target sequence and those microbeads for the unknown target sequence. This may be done by analysis of X-ray fluorescence characteristic of different metal elements which label the different reporter molecules. It then is an easy procedure to calibrate the assay of unknown target sequences against the known target sequence assay.

The use of a control target sequence can also be used to compensate for variations in the number of reporter molecules which may be linked to a single microbead. The microbeads are prepared so as to substantially have a single reporter molecule per single microbead. When more than one reporter molecule is linked to a single microbead, more than a single target sequence may cause disassociation of the reporter molecules linked to the single bead. A count of microbeads then will give a lower result for an indication of target sequence quantity than would be correct. This can readily be compensated by use of a control system in which the reporter molecules are linked to microbeads in the same way as those used for assay of the unknown target sequence. Alternatively, if the number of reporter molecules linked to a single microbead is known, the assay can be calibrated accordingly.

Employing X-ray fluorescence analysis for the detection step also makes it possible to simultaneously assay a plurality of different unknown target sequences. When this is to be done, the different kinds of target sequences in the sample of genetic material are assayed by admixing in the aqueous medium different varieties of hybridized nucleotide duplexes. Each different duplex variety should be comprised of first of all, a different type of genetic probe, whereby each type is capable of hybridizing with a specific different kind of target sequence, and secondly a reporter molecule linked to a differently labelled microbead, with the microbeads of different duplex varieties having different metal element labels. After introduction of the genetic material into the aqueous medium, reporter molecules having microbeads corresponding to the particular individual target sequences will be disassociated. They are simultaneously and separately detected by detecting the labels of the differently labelled microbeads by analysis of X-ray fluorescence characteristic of each different metal element.

One of the main features of the invention, the homogeneous hybridization of the target sequence in the aqueous medium without affixing it to a solid, is maintained in a further embodiment of the present invention. In this embodiment the genetic probe is labelled and acts as a reporter in a negatively correlated assay whereby a separate reporter molecule may be omitted.

In this alternative embodiment, genetic material including a target sequence is admixed in an aqueous medium under predetermined assay conditions with weakly hybridized nucleotide duplexes of (a) a genetic probe for said target sequence, the genetic probe being linked to a labelled microbead, and (b) a homopolymer molecule, the genetic probe being capable of hybridizing more strongly with the target sequence than with the homopolymer under the predetermined assay conditions. The target sequences then hybridize with genetic probes of the probe-homopolymer duplexes to form target-probe duplexes and the labelled microbeads carried by the genetic probes of the formed target-probe duplexes are disassociated from said homopolymers. Remaining probe-homopolymer duplexes are segregated from target-probe duplexes and the labels of the labelled microbeads in the remaining probe-homopolymer duplexes are detected. What is measured is the decrease from a count of labels in the initially provided probe-homopolymer duplexes.

A weak hybridization of the initially provided duplex and a stronger hybridization with the duplex containing a target sequence is provided in the same way as described above for the first embodiment. To accomplish this, the target sequence comprises a characteristic sense sequence and the genetic probe comprises (1) an antisense sequence complementary to the characteristic sense sequence of said target sequence and (2) a hybridizable sequence adapted to hybridize with the homopolymer. A number of nucleotides which are capable of forming base pairs with the homopolymer are comprised by said hybridizable sequence or by both said hybridizable sequence and the antisense sequence of the genetic probe.

The number of base pair-forming nucleotides should be sufficient under the predetermined assay conditions for the genetic probe and homopolymer of the probe-homopolymer duplex to remain hybridized in the absence of the target sequences, but insufficient for remaining hybridized under the reaction conditions when target sequences are present in the aqueous medium, due to blocking of some of the base pair-forming nucleotides by the target sequence. Preferably, the base pair-forming nucleotides are comprised by both the hybridizable sequence and the antisense sequence of the genetic probe, and the base pair-forming nucleotides of the antisense sequence are located adjacent to the hybridizable sequence. The homopolymers may be composed of X nucleotides and the base pair-forming nucleotides of the genetic probe of Y nucleotides in combinations as described hereinabove.

Conditions of the assay such as temperature or pH are predetermined so that the genetic probe forms a weakly hybridized duplex with the homopolymer and a strongly hybridized duplex with the target sequence. Preferably the assay is conducted at a temperature just below the melting temperature of the duplex of the genetic probe and the homopolymer.

To check the assay result, if desired, the assay is conducted at a temperature just below the melting temperature of the duplex of the genetic probe and the homopolymer and after first measuring a decrease in the count of labels of probe-homopolymer duplexes, the assay temperature is lowered to a temperature below the melting temperature of a duplex formable between (a) the base pair-forming nucleotides comprised by the hybridizable sequence of said genetic probe and (b) said homopolymer, and the labels of labelled microbeads in probe-homopolymer duplexes are detected to measure an increase of label count over the count of the first measurement. The microbeads are labelled in the same way as described above, and X-ray fluorescence analysis is also the preferred detection procedure.

It is preferred that the homopolymer of the probe-homopolymer duplexes should be linked to a solid substrate in a fixed location. This may be part of the container for the aqueous medium, as described above. This permits segregation of remaining probe-homopolymer duplexes from target-probe duplexes by use of a narrow solid angle X-ray fluorescence detector as described above. Alternatively, the aqueous medium may be removed from the solid substrate which optionally is washed to remove target-probe duplexes therefrom and thus eliminate noise.

In the alternative embodiment of the invention, a known target sequence can be employed as a control, and multiple unknown target sequences may be assayed in a similar way to that described above.

In the case of use of a control, the method comprises also admixing in the aqueous medium as a control a known quantity of a known target sequence and additional weakly hybridized nucleotide duplexes. The additional duplexes are of (c) a genetic probe for the known target and (d) a homopolymer molecule, said genetic probe (c) being linked to a labelled microbead, said microbead being labelled with a metal element different from the label of the genetic probe for the unknown target. The genetic probe (c) for the known target is capable of hybridizing more strongly with said known target sequence than with the homopolymer (d) under predetermined assay conditions. There is simultaneously and separately detected: I. the labels of the labelled microbeads in the remaining probe for the unknown target (a)-homopolymer (b) duplexes and II. the labels of the labelled microbeads in the remaining probe (c)-homopolymer (d) duplexes, by analysis of X-ray fluorescence characteristic of said different metal elements, and the assay of the target sequences is calibrated against the assay of the control.

For a simultaneous determination of multiple target sequences, wherein said genetic material includes different kinds of target sequences to be assayed, said different kinds are assayed by admixing in the aqueous medium different varieties of weakly hybridized nucleotide duplexes, each different duplex variety being comprised of (a) a different type of genetic probe, each type being capable of hybridizing with a particular one of said different kinds of target sequences to be assayed and each type of genetic probe being linked to a differently labelled microbead, and (b) a homopolymer molecule, the microbeads of the different duplex varieties having different metal element labels; and simultaneously and separately detecting the labels of the differently labelled microbeads in the remaining probe-homopolymer duplexes by analysis of X-ray fluorescence characteristic of each different metal element.

In accordance with the invention, a kit is provided. The kit may comprise components which enable an assay in accordance with either of the alternative embodiments to be performed.

In one embodiment, the essential components of the kit are contained in an aqueous medium together or in separate portions of the medium, these components being:

A. weakly hybridized nucleotide duplexes of (i) a genetic probe for the target sequence and (ii) a reporter molecule linked to a labelled microbead; and

B. a plurality of nucleotide-comprising homopolymers capable of selectively hybridizing with the reporter molecules (ii) when said reporter molecules are not hybridized with the genetic probes (i).

The characteristics of the components of said kit are as described above in connection with the first embodiment of the method of the invention.

The kit may further comprise a solid substrate to which the homopolymers are linked, and this may be part of the container in which the aqueous medium may be contained.

In an embodiment of this kit, whereby different kinds of target sequences may be simultaneously assayed, component A. additionally comprises different varieties of hybridized nucleotide duplexes, each different duplex variety being composed of (i) a different type of genetic probe, each type being capable of hybridizing with a particular one of said different kinds of target sequences to be assayed, and (ii) a reporter molecule linked to a differently labelled microbead, the microbeads of different duplex varieties having different metal element labels, said different duplex varieties being contained in the same aqueous medium or in separate portions thereof.

An embodiment of the kit also provides for use of a control, wherein the kit further includes:

C. a known quantity of a known target sequence as a control, contained in a portion of the aqueous medium which is separate from the aqueous medium containing component A.; and

D. additional weakly hybridized nucleotide duplexes, said additional duplexes being of (iii) a genetic probe for said known target sequence and (iv) a reporter molecule linked to a labelled microbead, said microbead being labelled with a metal element different from the label of the reporter molecule (ii), said genetic probe (iii) being capable of hybridizing more strongly with said known target sequence than with said reporter molecule (iv) under predetermined assay conditions.

A further embodiment of the kit is useful for carrying out the above-described alternative method. This kit comprises an aqueous medium containing component (A) which comprises weakly hybridized nucleotide duplexes of (a) a genetic probe for said target sequence, said genetic probe being linked to a labelled microbead, and (b) a homopolymer molecule, said genetic probe being capable of hybridizing more strongly with the target sequence than with the homopolymer under predetermined assay conditions.

The characteristic of the components of this kit are as described above in connection with the alternative embodiment of the assay method.

This alternative kit optionally may further comprise a solid substrate to which the homopolymers are linked, and the solid can be part of a container for the aqueous medium.

The kit may be adapted for assaying different kinds of target sequences, in which case component (A) additionally comprises different varieties of hybridized nucleotide duplexes, each different duplex variety being comprised of (a) a different type of genetic probe, each type being capable of hybridizing with a particular one of said different kinds of target sequences to be assayed and each type of genetic probe being linked to a differently labelled microbead, and (b) a homopolymer molecule, the microbeads of the different duplex varieties having different metal element labels, said different duplex varieties being contained in the same aqueous medium or in separate portions thereof.

Where a control target sequence is to be assayed the kit further includes: B. a known quantity of a known target sequence as a control, contained in a portion of the aqueous medium which is separate from the aqueous medium containing component A.; and

C. additional weakly hybridized nucleotide duplexes, said additional duplexes being of (c) a genetic probe for said known target sequence and (d) a homopolymer molecule, said genetic probe (c) being linked to a labelled microbead, said microbead being labelled with a metal element different from the label of the genetic probe (a) for the unknown target sequence, and said genetic probe (c) being capable of hybridizing more strongly with said known target sequence than with said homopolymer (d) under predetermined assay conditions.

The assay of the present invention is advantageous since it can be accomplished quickly and readily, with great sensitivity of measurement. There is no loss of target sequence binding sites through binding to a solid substrate. The method of the invention eliminates a number of steps required in the prior art such as centrifugation, and in most cases washing steps. By use of microbeads, the detection is highly sensitive because of the amplification factor of the numerous labels carried in or on the microbead. The need to handle and dispose of radioactive materials can be eliminated by choosing one of the other detection procedures such as the preferred X-ray fluorescence procedure. The latter procedure leads to the further advantage of enabling simultaneous assay with a control and/or simultaneous assay of a number of different unknown target sequences.

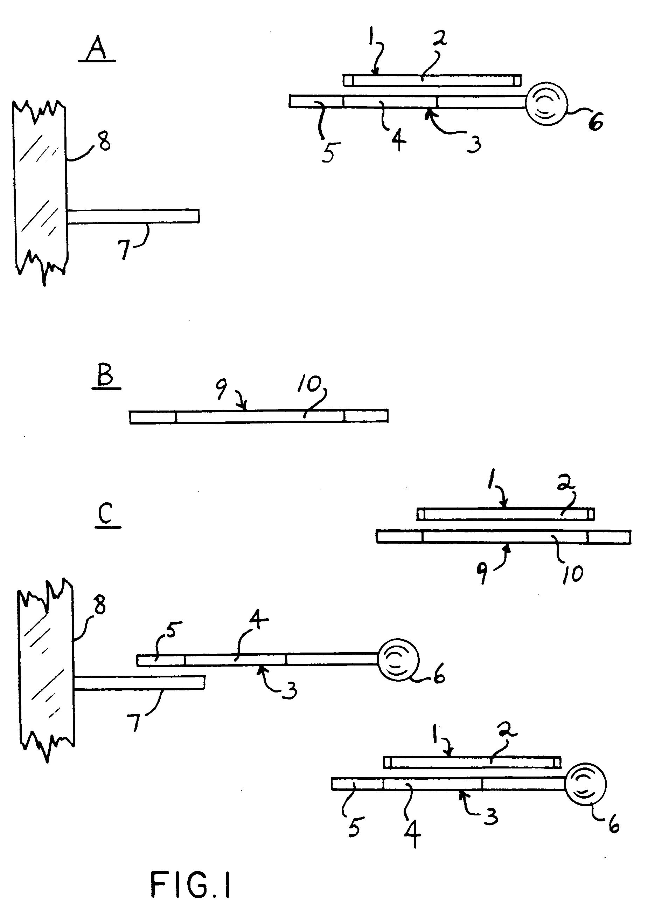

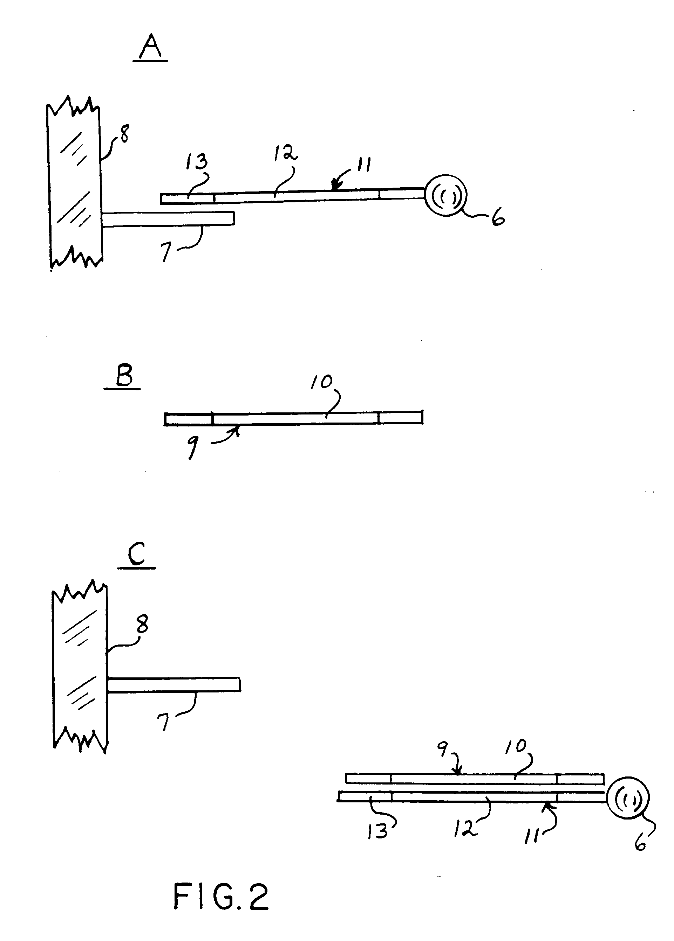

Examples of the novel method are illustrated in Figures 1 and 2.

Figure 1 illustrates the components in the aqueous medium during three stages of the assay according to the above first described embodiment of the method of the invention. The components are understood to be in an aqueous medium (not shown). A genetic probe 1 having an antisense sequence 2 forms a duplex with a reporter molecule 3. The reporter molecule 3 comprises a sense sequence 4, a hybridizable sequence 5 and a microbead 6 having labels (not shown). At this stage also in the medium are homopolymer sequence 7 linked to solid substrate 8. Stage B represents introduction of genetic material including unknown target sequence 9 having sense sequence 10.

Following the introduction of the target sequence of stage B, stage C depicts the resulting components in the aqueous medium. Reporter 3 has disassociated from its previous duplex with the probe 1, and homopolymer 7 forms a duplex by hybridization with the hybridizable sequence 5 and part of the sense sequence 4 of reporter 3, whereby microbead 6 becomes located at the location of solid substrate 8. Probe 1 has become disassociated from the previous duplex with the reporter molecule and forms a duplex with target sequence 9 by hybridization of the antisense sequence 2 of probe 1 with the sense sequence 10 of target 9. The medium further contains undisassociated duplexes of reporter molecule 3 and genetic probe 1.

Figure 2 illustrates the above-described alternative embodiment of the method of the invention, and indicates the components present in the aqueous medium (not shown) at stages A, B and C. Stage A represents the aqueous medium at the start of the assay before introduction of genetic material. The medium contains genetic probe 11 having an antisense sequence 12 and hybridizable sequence 13 as well as a microbead 6 which has labels (not shown). Homopolymer 7 which is linked to solid substrate 8 forms a duplex with probe 11 by hybridization with the hybridizable sequence 13 and part of the antisense sequence 12 of probe 11. Stage B represents the introduction into the medium of genetic material containing unknown target sequence 9 comprising sense sequence 10. Stage C represents the components present after introduction of the genetic material. The genetic probe 11 has disassociated from homopolymer 7 and has formed a new duplex between the sense sequence 10 of the target sequence and the antisense sequence 12 of the genetic probe. Microbead 6 is thereby carried with the duplex away from the location on solid substrate 8 of the homopolymer 7.

To simplify the figures, only a single one of each component is shown at each stage although it will be appreciated that a plurality of such components would ordinarily be present.

The following examples are provided to illustrate but not limit the invention. In the examples commercially available deoxynucleotide homopolyers are used. Details of linking homopolymers to substrates and of linking the reporter molecules to latex such as the microbeads can be found in the above-mentioned text of Maniatis, et al., "Molecule Cloning". Viral and cellular myc and ras DNA clones are commercially available. Appropriate DNA fragments are prepared in a known way. Various cell lines and primary cells known to express different levels of myc and ras sequences can be used for testing. Appropriate oncogene probes for ras and myc are commercially available.

The melting temperature ($T_m$) of DNA duplexes in many cases is known or can be calculated from the formula:

$$T_m = 80°C + 18.5 \ (\log \text{ concentration Na Cl}) + 58.4 \ (\% \text{ CG pairs}) - \left( \frac{820}{\text{number of base pairs}} \right)$$

Melting temperatures for DNA duplexes are known to be about as follows:

| number of base pairs | $T_m$ (°C) |
|---|---|
| 25 | 75 |
| 15 | 55 |
| 12 | 40 |
| 10 | 25 |

EXAMPLE I

Restriction fragments of genetic material can be assayed for the presence and quantity of ras and myc oncogenes. An aqueous medium is provided, within which are contained:

(1) homopolymer sequences of 20 C nucleotides linked to a glass substrate;

(2) duplexes of (a) a genetic probe having an antisense sequence for ras, said probe having 25 nucleotides, hybridized with (b) a reporter molecule having 8 G nucleotides in a hybridizable sequence and a sense sequence having 15 nucleotides with the GC to AT ratio being 1, and being complementary to nucleotides of said antisense sequence of the probe; the sense sequence of the reporter molecule comprises 3 G nucleotides located adjacent to the hybridizable sequence; the reporter molecule is linked to a microbead labelled with Cr atoms;

(3) duplexes which are the same as those of (2) above but wherein the antisense sequence of the genetic probe is for myc and the microbeads are labelled with Ti atoms.

The assay is conducted at about 50°C. Since the melting temperature for 15 base pairs is about 55°C, the above duplexes (2) and (3) remain in the medium as weak hybrids formed from 15 nucleotides of the reporter sense sequence and a like number of the genetic probe antisense sequence. The hybrid of 15 base pairs has a melting temperature just above the assay temperature.

A hybrid between the reporter molecule and the homopolymer does not form at the assay temperature because only 8 G nucleotides from the hybridizable sequence of the reporter molecule are available for forming base pairs with the homopolymer, and a hybrid of 8 GC pairs has a melting temperature of 35°C, much below the assay temperature.

Genetic restriction fragments including target ras and myc sequences are introduced into the aqueous medium. Since the antisense sequence of the genetic probe has 25 nucleotides, the genetic probes disassociate from the weakly hybridized probe-reporter duplexes and form more strongly hybridized duplexes with the target ras and myc sequences. Each disassociated probe-reporter duplex releases a reporter molecule linked to a microbead having a label specific to the target sequence which hybridized with the probe of that initial duplex.

The disassociated reporter molecules form duplexes by hybridization because upon release from the genetic probe, the reporter molecule contains 8 G nucleotides in its hybridizable sequence and an adjacent 3 more G nucleotides in its sense sequence which are no longer blocked by hybridization with the probe, making available a total of 11 G nucleotides for hybridization with the homopolymer. A homopolymer-reporter hybrid having 11 GC pairs is formed and has a melting temperature of about 63°C. which is above the temperature of the assay.

The microbeads of the homopolymer-reporter duplexes are fixed in location at the location of the glass substrate. Detection of the labels is done by X-ray fluorescence as described above. Fluorescent signals from Cr are correlated with quantity of ras and fluorescent signals from Ti are simultaneously obtained and correlated with quantity of myc.

EXAMPLE 2

Restriction fragments as described in Example 1 are assayed. An aqueous medium is provided, within which are contained:

(i) duplexes of:

(a) a homopolymer sequence having 20 C nucleotides linked at one end to a glass substrate, the homopolymer being hybridized with (b) a genetic probe having a hybridizable sequence containing 8 G nucleotides and a 25 nucleotide antisense sequence for ras, said antisense sequence is selected to contain 3 G nucleotides located at the end of the hybridizable sequence; the genetic probe is linked to a microbead labelled with Cr atoms;

10

(ii) duplexes which are the same as those of (i) above but wherein the antisense sequence of the genetic probe is for myc and the microbeads are labelled with Ti atoms.

The quantity of microbeads of each label type thus located at the location of the substrate is a standard known quantity, or the quantity is measured by X-ray fluorescence. The assay temperature is 45°C.

Each homopolymer-probe duplex is formed from a total of 11 GC pairs in the genetic probe hybridized with a like number of nucleotides in the homopolymer. Since the melting temperature for 11 GC base pairs is 63°C, which is above the assay temperature of 45°C, the homopolymer-probe duplexes (i) and (ii) remain in the medium as hybrids.

Genetic restriction fragments including target ras and myc sequences are introduced into the aqueous medium. Since the antisense sequence of the genetic probe has 25 nucleotides, it preferentially hybridizes with the target sequence, the target thereby blocking the 3 G nucleotides of the antisense sequence which are adjacent to the hybridizable sequence of the genetic probe. This reduces the G nucleotides of the genetic probe available for hybridizing with the homopolymer to the 8 G nucleotides of the hybridizable sequence. The melting temperature for a duplex of 8 GC pairs is about 35°C, which is below the assay temperature. Therefore, the genetic probe is released from the homo-polymer, carries away the labelled microbead and is pre-sent in the medium as a probe-target duplex.

The labels of microbeads of the homopolymer-probe duplexes remaining fixed in location at the location of the glass substrate are detected by X-ray fluorescence as described above. The decrease of fluorescent signals from Cr is correlated with quantity of ras and the decrease of fluorescent signals from Ti is simultaneously measured and correlated with quantity of myc.

## Claims

1. A method for assaying genetic sequences, which comprises:

admixing in an aqueous medium under predetermined assay conditions (1) genetic material which includes a sequence to be assayed (target sequence) and (2) weakly hybridized nucleotide duplexes of (i) a genetic probe for said target sequence, wherein the target sequence comprises a characteristic sense sequence and the genetic probe comprises an antisense sequence complementary to the characteristic sense sequence of said target sequence, and (ii) a reporter molecule linked to labelled microbead, said genetic probe being capable of hybridizing more strongly with said target sequence than with said reporter molecule under said predetermined assay conditions;

whereby target sequences hybridize with genetic probes of said duplexes and, correspondingly, reporter molecules disassociate from said probe-reporter duplexes;

differentiating disassociated reporter molecules from undisassociated probe-reporter duplexes;

and detecting the labels of the microbeads which are linked to the differentiated, disassociated reporter molecules.

2. A method according to claim 1, wherein the reporter molecule comprises a sense sequence which is shorter than the target sense sequence and is complementary to part of the genetic probe antisense sequence.

3. A method according to claim 1 or 2, wherein conditions of the assay selected from temperature and pH are predetermined so that the genetic probe forms a weakly hybridized duplex with the reporter molecule and a strongly hybridized duplex with the target sequence.

4. A method according to claim 3, wherein the assay is conducted at a temperature just below the melting temperature of the duplex of the genetic probe and the reporter molecule.

5. A method according to claim 2, further comprising providing in said aqueous medium a number of nucleotide polymers which selectively hybridize with disassociated reporter molecules, for differentiating said disassociated reporter molecules from undisassociated reporter molecules.

6. A method according to claim 5, wherein the nucleotide polymers are nucleotide-comprising homo-polymer molecules.

7. A method according to claim 6, wherein the reporter molecule comprises a hybridizable sequence adapted to hybridize with said homopolymer, and further a number of nucleotides which are capable of forming base pairs with said homopolymer are comprised by said hybridizable sequence or by both said hybridizable sequence and the sense sequence of the reporter molecule.

8. A method according to claim 7, wherein the number of base pair-forming nucleotides is sufficient under the predetermined assay conditions for the formation of a reporter-homopolymer duplex between said hybridizable sequence and said homopolymer when the reporter molecule is disassociated from the genetic probe, and is insufficient under the reaction conditions to form said reporter-homopolymer duplex when the reporter molecule is hybridized with the genetic probe due to blocking of some of said base pair-forming nucleotides by said genetic probe.

9. A method according to claim 8, wherein the base pair-forming nucleotides are comprised by both the hybridizable sequence and the sense sequence of the reporter molecule, and the base pair-forming nucleotides of said sense sequence are located adjacent to said hybridizable sequence.

10. A method according to claim 8, wherein the homopolymers are linked to a solid substrate on which disassociated reporters are collected by formation of reporter-homopolymer duplexes.

11. A method according to claim 8, wherein each homopolymer molecule is linked to a magnetically labelled microbead, disassociated labelled reporter molecules are differentiated from undisassociated probe-reporter duplexes by forming reporter-homopolymer duplexes having magnetic labels and collecting the reporter-homopolymer duplexes by magnetic attraction to a desired location, and detecting the labels of micro-beads linked to reporter molecules at said location.

12. A method according to any of claims 1 to 10, wherein said micro-beads are labelled with colored compounds, compounds which are fluorescent under ultraviolet illumination, radioactive tracers or with metal elements or compounds detectable by X-ray fluorescence.

13. A method according to claim 12, wherein the microbeads are labelled with metal elements or compounds and the labelled microbeads linked to disassociated reporter sequences are detected by X-ray fluorescence analysis.

14. A method according to claim 10, wherein the microbeads are labelled with metal elements or compounds and the labelled microbeads linked to disassociated reporter molecules are detected by X-ray fluorescence analysis utilizing a source of X-rays or electrons for irradiating disassociated reporter molecules collected on the solid substrate and an X-ray fluorescence detector capable of viewing a narrow solid angle, and the fixed location of the homopolymers on said solid substrate is selected in relation to said detector and said aqueous medium so that said detector essentially views fluorescent X-rays emitted by said collected disassociated reporter molecules without viewing a significant number of fluorescent X-rays emitted by undisassociated reporter molecules in said aqueous medium.

15. A method according to claim 13 or 14, further comprising also admixing in the aqueous medium as a control a known quantity of a known target sequence and additional weakly hybridized nucleotide duplexes, said additional duplexes being of (iii) a genetic probe for said known target sequence and (iv) a reporter molecule linked to a labelled microbead, said microbead being labelled with a metal element different from the label of the reporter molecule (ii), said genetic probe (iii) being capable of hybridizing more strongly with said known target sequence than with said reporter molecule (iv) under said predetermined assay conditions;

simultaneously and separately detecting (A) the labels of the microbeads which are linked to reporter molecules (ii) having been disassociated from the genetic probe (i) for the unknown target sequence and (B) the labels of the microbeads which are linked to reporter molecules (iv) having been disassociated from the genetic probe (iii) for the known target sequence, by analysis of X-ray fluorescence characteristic of said different metal elements;

and calibrating the assay of the unknown target sequences against the assay of the known target sequence.

16. A method according to claim 13, 14 or 15, wherein:

said genetic material includes different kinds of target sequences to be assayed, and said different kinds of target sequences are assayed by admixing in the aqueous medium different varieties of hybridized nucleotide duplexes, each different duplex variety being comprised of (i) a different type of

EP 0 330 185 B1

genetic probe, each type being capable of hybridizing with a particular one of said different kinds of target sequences to be assayed, and (ii) a reporter molecule linked to a differently labelled microbead, the microbeads of different duplex varieties having different metal element labels;

and simultaneously and separately detecting the labels of the differently labelled microbeads by analysis of X-ray fluorescence characteristic of each different metal element.

17. A method for assaying genetic sequences, which comprises:

admixing in an aqueous medium under predetermined assay conditions (I) genetic material which includes a sequence to be assayed (target sequence) and (II) weakly hybridized nucleotide duplexes of (a) a genetic probe for said target sequence, said genetic probe being linked to a labelled microbead, and (b) a homopolymer molecule, wherein the target sequence comprises a characteristic sense sequence and the genetic probe comprises (1) an antisense sequence complementary to the characteristic sense sequence of said target sequence and (2) a hybridizable sequence adapted to hybridize with said homopolymer, said genetic probe being capable of hybridizing more strongly with said target sequence than with said homopolymer under said predetermined assay conditions;

whereby target sequences hybridize with genetic probes of the probe-homopolymer duplexes to form target-probe duplexes and the labelled microbeads carried by the genetic probes of the formed target-probe duplexes are disassociated from said homopolymers;

segregating the remaining probe-homopolymer duplexes from target-probe duplexes;

and detecting the labels of the labelled microbeads in the remaining probe-homopolymer duplexes to measure the decrease from a count of labels in the initially provided probe-homopolymer duplexes.

18. A method according to claim 17, wherein the homopolymers of the probe-homopolymer duplexes are linked to a solid substrate in a fixed location.

19. A method according to claim 17 or 18, wherein a number of nucleotides which are capable of forming base pairs with said homopolymer are comprised by said hybridizable sequence or by both said hybridizable sequence and the antisense sequence of the genetic probe.

20. A method according to claim 19, wherein the number of base pair-forming nucleotides is sufficient under the predetermined assay conditions for the genetic probe and homopolymer of said probe-homopolymer duplex to remain hybridized in the absence of said target sequences, and is insufficient for remaining hybridized under the reaction conditions when target sequences are present in the aqueous medium, due to blocking of some of said base pair-forming nucleotides by said target sequence.

21. A method according to claim 20, wherein the base pair-forming nucleotides are comprised by both the hybridizable sequence and the antisense sequence of the genetic probe, and the base pair-forming nucleotides of said antisense sequence are located adjacent to said hybridizable sequence.

22. A method according to any of claims 17 to 21, wherein conditions of the assay selected from temperature and pH are predetermined so that the genetic probe forms a weakly hybridized duplex with the homopolymer and a strongly hybridized duplex with the target sequence.

23. A method according to claim 22, wherein the assay is conducted at a temperature just below the melting temperature of the duplex of the genetic probe and the homopolymer.

24. A method according to claim 23, wherein after first measuring a decrease in the count of labels of probe-homopolymer duplexes, the assay temperature is lowered to a temperature below the melting temperature of a duplex formable between (a) the base pair-forming nucleotides comprised by the hybridizable sequence of said genetic probe and (b) said homopolymer, and the labels of labelled microbeads in probe-homopolymer duplexes are detected to measure an increase of label count over the count of the first measurement.

25. A method according to any of claims 17 to 24, wherein said micro-beads are labelled with colored compounds, compounds which are fluorescent under ultraviolet illumination, radioactive tracers or with metal elements or compounds detectable by X-ray fluorescence.

13

26. A method according to claim 25, wherein the microbeads are labelled with metal elements or compounds and the labelled microbeads in the probe-homopolymer duplexes are detected by X-ray fluorescence analysis.

27. A method according to claim 18, wherein the microbeads are labelled with metal elements or compounds and the labelled microbeads linked to probe-homopolymer duplexes are detected by X-ray fluorescence analysis utilizing a source of X-rays or electrons for irradiating the probe-homopolymer duplexes collected on said solid substrate and an X-ray fluorescence detector capable of viewing a narrow solid angle, and the fixed location of said solid substrate is selected in relation to said detector and said aqueous medium so that said detector essentially views fluorescent X-rays emitted by said collected probe-homopolymer duplexes without viewing a significant number of fluorescent X-rays emitted by target-probe duplexes in said aqueous medium.

28. A method according to claim 26 or 27, further comprising also admixing in the aqueous medium as a control a known quantity of a known target sequence and additional weakly hybridized nucleotide duplexes, said additional duplexes being of (c) a genetic probe for said known target sequence and (d) a homopolymer molecule, said genetic probe (c) being linked to a labelled microbead, said microbead being labelled with a metal element different from the label of the genetic probe (a) for the unknown target, and said genetic probe (c) being capable of hybridizing more strongly with said known target sequence than with said homopolymer (d) under said predetermined assay conditions;

simultaneously and separately detecting I. the labels of the labelled microbeads in the remaining probe (a) -homopolymer (b) duplexes and II. the labels of the labelled microbeads in the remaining probe (c)-homopolymer (d) duplexes, by analysis of X-ray fluorescence characteristic of said different metal elements;

and calibrating the assay of the target sequences against the assay of the control.

29. A method according to claim 26, 27 or 28, wherein said genetic material includes different kinds of target sequences to be assayed, and said different kinds of target sequences are assayed by admixing in the aqueous medium different varieties of weakly hybridized nucleotide duplexes, each different duplex variety being comprised of (a) a different type of genetic probe, each type being capable of hybridizing with a particular one of said different kinds of target sequences to be assayed and each type of genetic probe being linked to a differently labelled microbead, and (b) a homopolymer molecule, the microbeads of the different duplex varieties having different metal element labels;

and simultaneously and separately detecting the labels of the differently labelled microbeads in the remaining probe-homopolymer duplexes by analysis of X-ray fluorescence characteristic of each different metal element.

30. A kit for carrying out the method of claim 1 for assaying genetic sequences in an aqueous medium containing genetic material which includes a genetic target sequence to be assayed, said kit comprising an aqueous medium containing together in said medium or in separate portions of the medium the following components:

A. weakly hybridized nuclcotide duplexes of (i) a genetic probe for said target sequence and (ii) a reporter molecule linked to a labelled microbead; and

B. a plurality of nucleotide-comprising homopolymers capable of selectively hybridizing with the reporter molecules (ii) when said reporter molecules are not hybridized with the genetic probes (i).

31. A kit according to claim 30, wherein said target Sequence, genetic probe, reporter molecule, labelled microbead and nucleotide-comprising homopolymers are as defined in any of claims 2, 7 to 9 or 11 to 13.

32. A kit according to claim 30 or 31, further comprising a solid substrate to which the homopolymers are linked.

33. A kit according to claim 30, 31 or 32, for assaying different kinds of target sequences, wherein component A. additionally comprises different varieties of hybridized nucleotide duplexes, each different duplex variety being composed of (i) a different type of genetic probe, each type being capable of hybridizing with a particular one of said different kinds of target sequences to be assayed, and (ii) a reporter molecule linked to a differently labelled microbead, the microbeads of different duplex varieties

14

having different metal element labels, said different duplex varieties being contained in the same aqueous medium or in separate portions thereof.

34. A kit according to any of claims 30 to 33, further including

C. a known quantity of a known target sequence as a control, contained in a portion of the aqueous medium which is separate from the aqueous medium containing component A.; and

D. additional weakly hybridized nucleotide duplexes, said additional duplexes being of (iii) a genetic probe for said known target sequence and (iv) a reporter molecule linked to a labelled microbead, said microbead being labelled with a metal element different from the label of the reporter molecule (ii), said genetic probe (iii) being capable of hybridizing more strongly with said known target sequence than with said reporter molecule (iv) under predetermined assay conditions.

35. A kit for carrying out the method of claim 17, for assaying genetic sequences in an aqueous medium containing genetic material which includes a genetic target sequence to be assayed, said kit comprising an aqueous medium containing component (A) which comprises weakly hybridized nucleotide duplexes of (a) a genetic probe for said target sequence, said genetic probe being linked to a labelled microbead, and (b) a homopolymer molecule, said genetic probe being capable of hybridizing more strongly with said target sequence than with said homopolymer under predetermined assay conditions.

36. A kit according to claim 35, wherein said target sequence, genetic probe, labelled microbead and homopolymer molecule are as defined in any of claims 19 to 21, 25 or 26.

37. A kit according to claim 35 or 36, further comprising a solid substrate to which the homopolymers are linked.

38. A kit according to claim 35, 36 or 37, for assaying different kinds of target sequences, wherein component (A) additionally comprises different varieties of hybridized nucleotide duplexes, each different duplex variety being comprised of (a) a different type of genetic probe, each type being capable of hybridizing with a particular one of said different kinds of target sequences to be assayed and each type of genetic probe being linked to a differently labelled microbead, and (b) a homopolymer molecule, the microbeads of the different duplex varieties having different metal element labels, said different duplex varieties being contained in the same aqueous medium or in separate portions thereof.

39. A kit according to any of claims 35 to 38, further including

B. a known quantity of a known target sequence as a control, contained in a portion of the aqueous medium which is separate from the aqueous medium containing component A.; and

C. additional weakly hybridized nucleotide duplexes, said additional duplexes being of (c) a genetic probe for said known target sequence and (d) a homopolymer molecule, said genetic probe (c) being linked to a labelled microbead, said microbead being labelled with a metal element different from the label of the genetic probe (a) for the unknown target sequence, and said genetic probe (c) being capable of hybridizing more strongly with said known target sequence than with said homopolymer (d) under predetermined assay conditions.

**Patentansprüche**

1. Verfahren zur Analyse von Gensequenzen, umfassend die folgenden Schritte:

Beimischen in ein wäßriges Medium unter vorbestimmten Analysebedingungen (1) genetisches Material, welches eine zu analysierende Sequenz (Zielsequenz) enthält, und (2) schwach hybridisierte Nucleotiddubletts aus (i) einer genetischen Probe für die Zielsequenz, wobei die Zielsequenz eine charakteristische Sinnsequenz umfaßt, und die genetische Probe eine zu der charakteristischen Sinnsequenz der Zielsequenz komplementäre Antisinnsequenz umfaßt, und (ii) einem mit einem markierten Mikrokügelchen verbundenen Reportermolekül, wobei die genetische Probe in der Lage ist, unter den vorbestimmten Analysebedingungen mit der Zielsequenz stärker zu hybridisieren als mit dem Reportermolekül;

so daß Zielsequenzen mit genetischen Proben der Dubletts hybridisieren und dementsprechend Reportermoleküle von den Probe-Reporter-Dubletts dissoziieren;

Unterscheiden der dissoziierten Reportermoleküle von undissoziierten Probe-Reporter-Dubletts;

und Erfassen der Markierungen der Mikrokügelchen, die mit den differenzierten, dissoziierten Reportermolekülen verbunden sind.

2. Verfahren nach Anspruch 1, dadurch gekennzeichnet, daß das Reportermolekül eine Sinnsequenz umfaßt, die kürzer ist als die Zielsequenz und zu dem Teil der Antisinnsequenz der genetischen Probe komplementär ist.

3. Verfahren nach Anspruch 1 oder 2, dadurch gekennzeichnet, daß die anhand von Temperatur und pH-Wert ausgewählten Analysebedingungen so vorbestimmt sind, daß die genetische Probe ein schwach hybridisiertes Dublett mit dem Reportermolekül und ein stark hybridisiertes Dublett mit der Zielsequenz bildet.

4. Verfahren nach Anspruch 3, dadurch gekennzeichnet, daß die Analyse bei einer Temperatur knapp unter der Schmelztemperatur des Dubletts aus der genetischen Probe und dem Reportermolekül durchgeführt wird.

5. Verfahren nach Anspruch 2, bei dem ferner in dem wäßrigen Medium eine Anzahl von Nucleotidpolymeren vorgesehen wird, die wahlweise mit dissoziierten Reportermolekülen hybridisieren, um die dissoziierten Reportermoleküle von undissoziierten Reportermolekülen zu unterscheiden.

6. Verfahren nach Anspruch 5, dadurch gekennzeichnet, daß die Nucleotidpolymere nucleotidhaltige Homopolymermoleküle sind.

7. Verfahren nach Anspruch 6, dadurch gekennzeichnet, daß das Reportermolekül eine hybridisierbare Sequenz umfaßt, die mit dem Homopolymer hybridisieren kann, und ferner eine Anzahl von Nucleotiden, die in der Lage sind, Basenpaare mit dem Homopolymer zu bilden, und in der hybridisierbaren Sequenz oder sowohl in der hybridisierbaren Sequenz als auch in der Sinnsequenz des Reportermoleküls enthalten sind.

8. Verfahren nach Anspruch 7, dadurch gekennzeichnet, daß die Anzahl basenpaarbildender Nucleotide unter den vorbestimmten Analysebedingungen ausreichend ist, um ein Reporter-Homopolymer-Dublett zwischen der hybridisierbaren Sequenz und dem Homopolymer zu bilden, wenn das Reportermolekül von der genetischen Probe dissoziiert ist, und unter den Reaktionsbedingungen nicht ausreichend ist, um das Reporter-Homopolymer-Dublett zu bilden, wenn das Reportermolekül mit der genetischen Probe hybridisiert ist, weil einige der basenpaarbildenden Nucleotide von der genetischen Probe blockiert werden.

9. Verfahren nach Anspruch 8, dadurch gekennzeichnet, daß die basenpaarbildenden Nucleotide sowohl in der hybridisierbaren Sequenz als auch in der Sinnsequenz des Reportermoleküls enthalten sind, und die basenpaarbildenden Nucleotide der Sinnsequenz neben der hybridisierbaren Sequenz liegen.

10. Verfahren nach Anspruch 8, dadurch gekennzeichnet, daß die Homopolymere mit einem festen Substrat verbunden sind, auf dem dissoziierte Reportermoleküle durch Bildung von Reporter-Homopolymer-Dubletts angesammelt werden.

11. Verfahren nach Anspruch 8, dadurch gekennzeichnet, daß jedes Homopolymermolekül mit einem magnetisch markierten Mikrokügelchen verbunden ist, daß dissoziierte markierte Reportermoleküle von undissoziierten Probe-Reporter-Dubletts unterschieden werden, indem Reporter-Homopolymer-Dubletts mit magnetischer Markierung gebildet werden und die Reporter-Homopolymer-Dubletts durch magnetische Anziehung an einem gewünschten Ort gesammelt werden, und indem die Markierungen der Mikrokügelchen erfaßt werden, die an dieser Stelle mit den Reportermolekülen verbunden sind.

12. Verfahren nach einem der Ansprüche 1 bis 10, dadurch gekennzeichnet, daß die Mikrokügelchen mit angefärbten Verbindungen markiert sind, nämlich mit Verbindungen, die unter UV-Bestrahlung fluoreszieren, mit radioaktiven Tracern oder mit Metallelementen oder -verbindungen, die mittels Röntgenfluoreszenz nachweisbar sind.

13. Verfahren nach Anspruch 12, dadurch gekennzeichnet, daß die Mikrokügelchen mit Metallelementen oder -verbindungen markiert sind, und daß die mit dissoziierten Reportersequenzen verbundenen

EP 0 330 185 B1

markierten Mikrokügelchen durch Röntgenfluoreszenzanalyse nachgewiesen werden.

14. Verfahren nach Anspruch 10, dadurch gekennzeichnet, daß die Mikrokügelchen mit Metallelementen oder -verbindungen markiert sind, und daß die mit dissoziierten Reportermolekülen verbundenen markierten Mikrokügelchen mittels Röntgenfluoreszenzanalyse unter Verwendung einer Röntgenstrahlen- oder Elektronenquelle, mit der dissoziierte Reportermoleküle bestrahlt werden, die sich auf dem festen Substrat angesammelt haben, und unter Verwendung eines Röntgenfluoreszenzdetektors, der einen schmalen Raumwinkel betrachten kann, nachgewiesen werden, und daß die feste Lage der Homopolymere auf dem festen Substrat derart in bezug auf den Detektor und das wäßrige Medium ausgewählt wird, daß der Detektor im wesentlichen fluoreszierende Röntgenstrahlen betrachtet, die von den angesammelten dissoziierten Reportermolekülen ausgesandt werden, ohne dabei eine signifikante Anzahl von fluoreszierenden Röntgenstrahlen zu betrachten, die von undissoziierten Reportermolekülen in dem wäßrigen Medium ausgesandt werden.

15. Verfahren nach Anspruch 13 oder 14, ferner umfassend die folgenden Schritte: als Kontrolle in das wäßrige Medium Beimischen einer bekannten Menge einer bekannten Zielsequenz und zusätzlicher schwach hybridisierter Nucleotiddubletts, wobei die zusätzlichen Dubletts aus (iii) einer genetischen Probe für die bekannte Zielsequenz und (iv) einem mit einem markierten Mikrokügelchen verbundenen Reportermolekül bestehen, wobei das Mikrokügelchen mit einem von der Markierung des Reportermoleküls (ii) verschiedenen Metallelement markiert ist, und die genetische Probe (iii) in der Lage ist, unter den vorbestimmten Analysebedingungen mit der bekannten Zielsequenz stärker zu hybridisieren als mit dem Reportermolekül (iv);
gleichzeitig und getrennt voneinander Erfassen (A) der Markierungen der Mikrokügelchen, die mit Reportermolekülen (ii) verbunden sind, die von der genetischen Probe (i) für die unbekannte Zielsequenz dissoziiert wurden, und (B) der Markierungen der Mikrokügelchen, die mit Reportermolekülen (iv) verbunden sind, die von der genetischen Probe (iii) für die bekannte Zielsequenz dissoziiert wurden, anhand der Analyse der Röntgenfluoreszenzcharakteristik der verschiedenen Metallelemente;
und Eichen der Analyse der unbekannten Zielsequenzen anhand der Analyse der bekannten Zielsequenz.

16. Verfahren nach Anspruch 13, 14 oder 15, dadurch gekennzeichnet, daß das genetische Material verschiedene Arten von zu analysierenden Zielsequenzen umfaßt, und die verschiedenen Arten von Zielsequenzen analysiert werden, indem dem wäßrigen Medium verschiedene Varietäten von hybridisierten Nucleotiddubletts beigemischt werden, wobei jede der verschiedenen Dublettvarietäten aus (i) einer anderen Art von genetischer Probe besteht, wobei jede Art in der Lage ist, mit einer bestimmten der verschiedenen Arten von zu analysierenden Zielsequenzen zu hybridisieren, und (ii) aus einem Reportermolekül besteht, das mit einem anders markierten Mikrokügelchen verbunden ist, wobei die Mikrokügelchen verschiedener Dublettvarietäten verschiedene Metallelementmarkierungen besitzen;
und indem gleichzeitig und getrennt voneinander die Markierungen der unterschiedlich markierten Mikrokügelchen anhand der Analyse der Röntgenfluoreszenzcharakteristik von jedem der verschiedenen Metallelemente erfaßt werden.

17. Verfahren zur Analyse von Gensequenzen, umfassend die folgenden Schritte:
Beimischen in ein wäßriges Medium unter vorbestimmten Analysebedingungen (I) genetisches Material, welches eine zu analysierende Sequenz (Zielsequenz) enthält, und (II) schwach hybridisierte Nucleotiddubletts aus (a) einer genetischen Probe für die Zielsequenz, wobei die genetische Probe mit einem markierten Mikrokügelchen verbunden ist, und (b) einem Homopolymermolekül, wobei die Zielsequenz eine charakteristische Sinnsequenz umfaßt, und die genetische Probe (1) eine zu der charakteristischen Sinnsequenz der Zielsequenz komplementäre Antisinnsequenz und (2) eine hybridisierbare Sequenz umfaßt, die mit dem Homopolymer hybridisieren kann, wobei die genetische Probe in der Lage ist, unter den vorbestimmten Analysebedingungen mit der Zielsequenz stärker zu hybridisieren als mit dem Homopolymer;
so daß die Zielsequenzen mit genetischen Proben der Probe-Homopolymer-Dubletts zu Ziel-Probe-Dubletts hybridisieren, und die von den genetischen Proben der entstandenen Ziel-Probe-Dubletts getragenen markierten Mikrokügelchen von den Homopolymeren dissoziiert werden;
Abtrennen der übrigen Probe-Homopolymer-Dubletts von den Ziel-Probe-Dubletts;
und Erfassen der Markierungen der markierten Mikrokügelchen in den übrigen probe-Homopolymer-Dubletts, um die Abnahme von einer bestimmten Zahl von Markierungen in den anfangs vorgesehenen

17

Probe-Homopolymer-Dubletts zu messen.

18. Verfahren nach Anspruch 17, dadurch gekennzeichnet, daß die Homopolymere der Probe-Homopolymer-Dubletts an einem festen Ort mit einem festen Substrat verbunden sind.

19. Verfahren nach Anspruch 17 oder 18, dadurch gekennzeichnet, daß eine Anzahl von Nucleotiden, die in der Lage sind, Basenpaare mit dem Homopolymer zu bilden, in der hybridisierbaren Sequenz oder sowohl in der hybridisierbaren Sequenz als auch in der Antisinnsequenz der genetischen Probe enthalten sind.

20. Verfahren nach Anspruch 19, dadurch gekennzeichnet, daß die Anzahl von basenpaarbildenden Nucleotiden unter den vorbestimmten Analysebedingungen für die genetische Probe und das Homopolymer des Proben-Homopolymer-Dubletts ausreicht, um in Abwesenheit der Zielsequenzen hybridisiert zu bleiben, und nicht ausreicht, um unter den Reaktionsbedingungen, in denen Zielsequenzen in dem wäßrigen Medium vorhanden sind, hybridisiert zu bleiben, weil einige der basenpaarbildenden Nucleotiden durch die Zielsequenz blockiert werden.

21. Verfahren nach Anspruch 20, dadurch gekennzeichnet, daß die basenpaarbildenden Nucleotiden sowohl in der hybridisierbaren Sequenz als auch in der Antisinnsequenz der genetischen Probe enthalten sind, und daß die basenpaarbildenden Nucleotiden der Antisinnsequenz neben der hybridisierbaren Sequenz liegen.

22. Verfahren nach einem der Ansprüche 17 bis 21, dadurch gekennzeichnet, daß die anhand von Temperatur und pH-Wert ausgewählten Analysebedingungen so vorbestimmt sind, daß die genetische Probe ein schwach hybridisiertes Dublett mit dem Homopolymer und ein stark hybridisiertes Dublett mit der Zielsequenz bildet.

23. Verfahren nach Anspruch 22, dadurch gekennzeichnet, daß die Analyse bei einer Temperatur knapp unter der Schmelztemperatur des Dubletts aus genetischer Probe und Homopolymer durchgeführt wird.

24. Verfahren nach Anspruch 23, dadurch gekennzeichnet, daß nachdem zunächst die Abnahme der Anzahl von Markierungen von Probe-Homopolymer-Dubletts gemessen wurde, die Analysetemperatur auf eine Temperatur unter der Schmelztemperatur eines Dubletts gesenkt wird, das gebildet werden kann zwischen (a) den in der hybridisierbaren Sequenz der genetischen Probe enthaltenen basenpaarbildenden Nucleotiden und (b) dem Homopolymer, und die Markierungen der markierten Mikrokügelchen in Probe-Homopolymer-Dubletts erfaßt werden, um eine Zunahme der Anzahl von Markierungen gegenüber der Anzahl aus der ersten Messung zu messen.

25. Verfahren nach einem der Ansprüche 17 bis 24, dadurch gekennzeichnet, daß die Mikrokügelchen mit angefärbten Verbindungen markiert werden, nämlich mit Verbindungen, die unter UV-Bestrahlung fluoreszieren, mit radioaktiven Tracern oder mit Metallelementen oder -verbindungen, die mittels Röntgenfluoreszenz nachweisbar sind.

26. Verfahren nach Anspruch 25, dadurch gekennzeichnet, daß die Mikrokügelchen mit Metallelementen oder -verbindungen markiert werden, und die markierten Mikrokügelchen in den Probe-Homopolymer-Dubletts mittels Röntgenfluoreszenzanalyse nachgewiesen werden.

27. Verfahren nach Anspruch 18, dadurch gekennzeichnet, daß die Mikrokügelchen mit Metallelementen oder -verbindungen markiert werden, und die mit den Probe-Homopolymer-Dubletts verbundenen markierten Mikrokügelchen mittels Röntgenfluoreszenzanalyse nachgewiesen werden, wobei eine Röntgenstrahlen- oder Elektronenquelle zur Bestrahlung der auf dem festen Substrat angesammelten Probe-Homopolymer-Dubletts sowie ein Röntgenfluoreszenzdetektor verwendet werden, der einen schmalen Raumwinkel betrachten kann, und daß die feste Lage des festen Substrats in bezug auf den Detektor und das wäßrige Medium so ausgewählt wird, daß der Detektor im wesentlichen fluoreszierende Röntgenstrahlen betrachtet, die von den angesammelten Probe-Homopolymer-Dubletts ausgesandt werden, ohne eine signifikante Anzahl von fluoreszierenden Röntgenstrahlen zu betrachten, die von den Ziel-Probe-Dubletts in dem wäßrigen Medium ausgesandt werden.

**28.** Verfahren nach Anspruch 26 oder 27, ferner umfassend die folgenden Schritte: als Kontrolle in das wäßrige Medium Beimischen einer bekannten Menge einer bekannten Zielsequenz und zusätzlicher schwach hybridisierter Nucleotiddubletts, wobei die zusätzlichen Dubletts aus (c) einer genetischen Probe für die bekannte Zielsequenz und (d) einem Homopolymermolekül bestehen, wobei die genetische Probe (c) mit einem markierten Mikrokügelchen verbunden ist, und das Mikrokügelchen mit einem Metallelement markiert ist, das von der Markierung der genetischen Probe (a) für das unbekannte Ziel verschieden ist, und wobei die genetische Probe (c) unter den vorbestimmten Analysebedingungen mit der bekannten Zielsequenz stärker hybridisieren kann als mit dem Homopolymer (d);
gleichzeitig und getrennt voneinander Erfassen (I.) der Markierungen der markierten Mikrokügelchen in den übrigen Probe(a)-Homopolymer(b)-Dubletts und (II.) der Markierungen der markierten Mikrokügelchen in den übrigen Probe(c)-Homopolymer(d)-Dubletts anhand einer Analyse der Röntgenfluoreszenzcharakteristik der verschiedenen Metallelemente;
und Eichen der Analyse der Zielsequenzen anhand der Analyse der Kontrollprobe.

**29.** Verfahren nach Anspruch 26, 27 oder 28, dadurch gekennzeichnet, daß das genetische Material verschiedene Arten von zu analysierenden Zielsequenzen umfaßt, und daß die verschiedenen Arten von Zielsequenzen dadurch analysiert werden, daß dem wäßrigen Medium verschiedene Varietäten von schwach hybridisierten Nucleotiddubletts beigemischt werden, wobei jede der verschiedenen Dublettvarietäten aus (a) einer anderen Art genetischer Probe besteht, wobei jede Art mit einer bestimmten der verschiedenen Arten von zu analysierenden Zielsequenzen hybridisieren kann, und jede Art genetischer Probe mit einem anders markierten Mikrokügelchen verbunden ist, und (b) aus einem Homopolymermolekül besteht, wobei die Mikrokügelchen der verschiedenen Dublettvarietäten unterschiedliche Metallelementmarkierungen besitzen;
und daß gleichzeitig und getrennt voneinander die Markierungen der unterschiedlich markierten Mikrokügelchen in den übrigen Probe-Homopolymer-Dubletts durch Analyse der Röntgenfluoreszenzcharakteristik von jedem der verschiedenen Metallelemente nachgewiesen werden.

**30.** Ausrüstung zur Durchführung des Verfahrens von Anspruch 1 zur Analyse von Gensequenzen in einem wäßrigen Medium, welches genetisches Material enthält, das eine zu analysierende genetische Zielsequenz umfaßt, wobei die Ausrüstung ein wäßriges Medium umfaßt, das in dem Medium oder in getrennten Teilen des Mediums die folgenden Komponenten enthält:
A. schwach hybridisierte Nucleotiddubletts aus (i) einer genetischen Probe für die Zielsequenz und (ii) einem Reportermolekül, das mit einem markierten Mikrokügelchen verbunden ist; und
B. eine Vielzahl von nucleotidhaltigen Homopolymeren, die in der Lage sind, wahlweise mit den Reportermolekülen (ii) zu hybridisieren, wenn die Reportermoleküle nicht mit den genetischen Proben (i) hybridisiert sind.

**31.** Ausrüstung nach Anspruch 30, dadurch gekennzeichnet, daß die Zielsequenz, genetische Probe, Reportermolekül, markierte Mikrokügelchen und Nucleotide enthaltenden Homopolymere der Definition nach einem der Ansprüche 2, 7 bis 9 oder 11 bis 13 entsprechen.

**32.** Ausrüstung nach Anspruch 30 oder 31, ferner umfassend ein festes Substrat, mit dem die Homopolymere verbunden sind.

**33.** Ausrüstung nach Anspruch 30, 31 oder 32 zur Analyse verschiedener Arten von Zielsequenzen, dadurch gekennzeichnet, daß die Komponente A. zusätzlich verschiedene Varietäten von hybridisierten Nucleotiddubletts enthält, wobei jede der verschiedenen Dublettvarietäten sich zusammensetzt aus (i) einer anderen Art genetischer Probe, wobei jede Art in der Lage ist, mit einer bestimmten der verschiedenen Arten von zu analysierenden Zielsequenzen zu hybridisieren, und (ii) einem Reportermolekül, das mit einem unterschiedlich markierten Mikrokügelchen verbunden ist, wobei die Mikrokügelchen der verschiedenen Dublettvarietäten unterschiedliche Metallelementmarkierungen besitzen, und die verschiedenen Dublettvarietäten in demselben wäßrigen Medium oder in getrennten Teilen desselben enthalten sind.

**34.** Ausrüstung nach einem der Ansprüche 30 bis 33, ferner umfassend:
C. eine bekannte Menge einer bekannten Zielsequenz als Kontrolle, die in einem Teil des wäßrigen Mediums enthalten ist, der von dem die Komponente A. enthaltenden wäßrigen Medium getrennt ist; und

EP 0 330 185 B1

D. zusätzliche schwach hybridisierte Nucleotiddubletts, wobei die zusätzlichen Dubletts aus (iii) einer genetischen Probe für die bekannte Zielsequenz und (iv) einem mit einem markierten Mikrokügelchen verbundenen Reportermolekül bestehen, wobei das Mikrokügelchen mit einem von der Markierung des Reportermoleküls (ii) verschiedenen Metallelement markiert ist, und die genetische Probe (iii) in der Lage ist, unter den vorbestimmten Analysebedingungen mit der bekannten Zielsequenz stärker zu hybridisieren als mit dem Reportermolekül (iv).

35. Ausrüstung zur Durchführung des Verfahrens von Anspruch 17 zur Analyse von Gensequenzen in einem wäßrigen Medium, welches genetisches Material enthält, das eine zu analysierende genetische Zielsequenz umfaßt, wobei die Ausrüstung ein die Komponente (A) enthaltendes wäßriges Medium umfaßt, das schwach hybridisierte Nucleotiddubletts aus (a) einer genetischen Probe für die Zielsequenz umfaßt, wobei die genetische Probe mit einem markierten Mikrokügelchen verbunden ist, und (b) einem Homopolymermolekül, wobei die genetische Probe in der Lage ist, unter den vorbestimmten Analysebedingungen stärker mit der Zielsequenz zu hybridisieren als mit dem Homopolymer.

36. Ausrüstung nach Anspruch 35, dadurch gekennzeichnet, daß die Zielsequenz, die genetische Probe, das markierte Mikrokügelchen und das Homopolymer der Definition nach einem der Ansprüche 19 bis 21, 25 oder 26 entsprechen.

37. Ausrüstung nach Anspruch 35 oder 36, ferner umfassend ein festes Substrat, mit dem die Homopolymere verbunden sind.

38. Ausrüstung nach Anspruch 35, 36 oder 37 zur Analyse verschiedener Arten von Zielsequenzen, dadurch gekennzeichnet, daß die Komponente (A) zusätzlich verschiedene Varietäten von hybridisierten Nucleotiddubletts enthält, wobei jede der verschiedenen Dublettvarietäten aus (a) einer anderen Art genetischer Probe besteht, wobei jede dieser Arten in der Lage ist, mit einer bestimmten der verschiedenen Arten von zu analysierenden Zielsequenzen zu hybridisieren, und jede Art von genetischer Probe mit einem anders markierten Mikrokügelchen verbunden ist, und (b) aus einem Homopolymermolekül besteht, wobei die Mikrokügelchen der verschiedenen Dublettvarietäten verschiedene Metallelementmarkierungen aufweisen, und die verschiedenen Dublettvarietäten in demselben wäßrigen Medium oder in getrennten Teilen desselben enthalten sind.

39. Ausrüstung nach einem der Ansprüche 35 bis 38, ferner umfassend:
B. eine bekannte Menge einer bekannten Zielsequenz als Kontrolle, die in einem Teil des wäßrigen Mediums enthalten ist, der von dem die Komponente (A) enthaltenden wäßrigen Medium getrennt ist; und
C. zusätzliche schwach hybridisierte Nucleotiddubletts, wobei die zusätzlichen Dubletts aus (c) einer genetischen Probe für die bekannte Zielsequenz und (d) einem Homopolymermolekül bestehen, und die genetische Probe (c) mit einem markierten Mikrokügelchen verbunden ist, wobei das Mikrokügelchen mit einem Metallelement markiert ist, das von der Markierung der genetischen Probe (a) für die unbekannte Zielsequenz verschieden ist, und wobei die genetische Probe (c) in der Lage ist, unter den vorbestimmten Analysebedingungen mit der bekannten Zielsequenz stärker zu hybridisieren als mit dem Homopolymer (d).

**Revendications**

1. Procédé pour tester des séquences génétiques, qui comprend :
le mélange dans un milieu aqueux, dans des conditions de test prédéterminées, (1) d'un matériel génétique qui comprend une séquence qui doit être testée (séquence cible) et (2) de duplex nucléotidiques faiblement hybridés constitués par (i) une sonde génétique pour ladite séquence cible, la séquence cible comprenant une séquence sens caractéristique et la sonde génétique comprenant une séquence anti-sens complémentaire de la séquence sens caractéristique de ladite séquence cible, et par (ii) une molécule reporteur liée à une microbille marquée, ladite sonde génétique étant capable de s'hybrider plus fortement avec ladite séquence cible qu'avec ladite molécule reporteur dans lesdites conditions de test prédéterminées ;
de sorte que les séquences cibles s'hybrident avec les sondes génétiques desdits duplex et, de manière correspondante, les molécules reporteurs se dissocient desdits duplex sonde-reporteur;
la différenciation des molécules reporteurs dissociées d'avec les duplex sonde - reporteur non

20

dissociés;

et la détection des marqueurs des microbilles qui sont liées aux molécules reporteurs dissociées différenciées.

2. Procédé selon la revendication 1, dans lequel la molécule reporteur comprend une séquence sens qui est plus courte que la séquence sens de la cible et qui est complémentaire d'une partie de la séquence anti-sens de la sonde génétique.

3. Procédé selon la revendication 1 ou 2, dans lequel les conditions du test choisies parmi la température et le pH sont prédéterminées de manière que la sonde génétique forme un duplex faiblement hybridé avec la molécule reporteur et un duplex fortement hybridé avec la séquence cible.

4. Procédé selon la revendication 3, dans lequel le test est réalisé à une température juste inférieure à la température de fusion du duplex de la sonde génétique et de la molécule reporteur.

5. Procédé selon la revendication 2, comprenant en outre la fourniture dans ledit milieu aqueux d'un certain nombre de polymères nucléotidiques qui s'hybrident sélectivement avec les molécules reporteurs dissociées, pour différencier lesdites molécules reporteurs dissociées des molécules reporteurs non dissociées.

6. Procédé selon la revendication 5, dans lequel les polymères nucléotidiques sont des molécules homopolymériques comprenant des nucléotides.

7. Procédé selon la revendication 6, dans lequel la molécule reporteur comprend une séquence hybridable conçue pour s'hybrider avec ledit homopolymère, et en outre un certain nombre de nucléotides qui sont capables de former des paires de bases avec ledit homopolymère sont constitués par ladite séquence hybridable ou par ladite séquence hybridable et par la séquence sens de la molécule reporteur.

8. Procédé selon la revendication 7, dans lequel le nombre de nucléotides formateurs de paires de bases est suffisant dans les conditions de test prédéterminées pour la formation d'un duplex reporteur-homopolymère entre ladite séquence hybridable et ledit homopolymère lorsque la molécule reporteur est dissociée de la sonde génétique, et est insuffisant dans les conditions réactionnelles pour former ledit duplex reporteur-homopolymère lorsque la molécule reporteur est hybridée avec la sonde génétique du fait du blocage de certains desdits nucléotides formateurs de paires de bases par ladite sonde génétique.

9. Procédé selon la revendication 8, dans lequel les nucléotides formateurs de paires de bases sont constitués par la séquence hybridable et par la séquence sens de la molécule reporteur, et les nucléotides formateurs de paires de bases de ladite séquence sens sont situés en position adjacente à ladite séquence hybridable.

10. Procédé selon la revendication 8, dans lequel les homopolymères sont liés à un substrat solide sur lequel les reporteurs dissociés sont recueillis par formation de duplex reporteur-homopolymère.

11. Procédé selon la revendication 8, dans lequel chaque molécule d'homopolymère est liée à une microbille marquée magnétiquement, les molécules reporteurs marquées dissociées sont différenciées des duplex sonde-reporteur non dissociés par formation de duplex reporteur-homopolymère ayant des marqueurs magnétiques et collecte des duplex reporteur-homopolymère par attraction magnétique jusqu'à un emplacement voulu, et détection des marqueurs des microbilles liées à des molécules reporteurs audit emplacement.

12. Procédé selon l'une quelconque des revendications 1 à 10, dans lequel lesdites microbilles sont marquées avec des composés colorés, des composés qui sont fluorescents en éclairement ultraviolet, des traceurs radioactifs ou avec des éléments ou composés métalliques détectables par fluorescence X.

**13.** Procédé selon la revendication 12, dans lequel les microbilles sont marquées avec des éléments ou composés métalliques et les microbilles marquées liées à des séquences reporteurs dissociées sont détectées par analyse de fluorescence X.

**14.** Procédé selon la revendication 10, dans lequel les microbilles sont marquées avec des éléments ou composés métalliques et les microbilles marquées liées à des molécules reporteurs dissociées sont détectées par analyse de fluorescence X à l'aide d'une source de rayons X ou d'électrons pour irradier les molécules reporteurs dissociées recueillies sur le substrat solide et d'un détecteur de fluorescence X capable d'observer un angle solide étroit, et l'emplacement fixé des homopolymères sur ledit substrat solide est choisi en fonction dudit détecteur et dudit milieu aqueux de manière que ledit détecteur observe essentiellement les rayons X fluorescents émis par lesdites molécules reporteurs dissociées recueillies sans observer un nombre sensible de rayons X fluorescents émis par les molécules reporteurs non dissociées dans ledit milieu aqueux.

**15.** Procédé selon la revendication 13 ou 14, comprenant en outre le mélange dans le milieu aqueux en tant que témoin, d'une quantité connue d'une séquence cible connue et de duplex nucléotidiques faiblement hybridés supplémentaires, lesdits duplex supplémentaires étant constitués par (iii) une sonde génétique pour ladite séquence cible connue et par (iv) une molécule reporteur liée à une microbille marquée, ladite microbille étant marquée avec un élément métallique différent du marqueur de la molécule reporteur (ii), ladite sonde génétique (iii) étant capable de s'hybrider plus fortement avec ladite séquence cible connue qu'avec ladite molécule reporteur (iv) dans lesdites conditions de test prédéterminées;

la détection simultanée et séparée (A) des marqueurs des microbilles qui sont liées à des molécules reporteurs (ii) qui ont été dissociées de la sonde génétique (i) pour la séquence cible inconnue et (B) des marqueurs des microbilles qui sont liées aux molécules reporteurs (iv) qui ont été dissociées de la sonde génétique (iii) pour la séquence cible connue, par analyse de la fluorescence X caractéristique desdits éléments métalliques différents;

et l'étalonnage du test des séquences cibles inconnues par rapport au test de la séquence cible connue.

**16.** Procédé selon la revendication 13, 14 ou 15, dans lequel:

ledit matériel génétique comprend différents types de séquences cibles qui doivent être testés, et lesdits types différents de séquences cibles sont testés par mélange dans le milieu aqueux de différentes variétés de duplex nucléotidiques hybridés, chaque variété différente de duplex étant constituée par (i) un type différent de sonde génétique, chaque type étant capable de s'hybrider avec un type particulier parmi lesdits types différents de séquences cibles qui doivent être testés et par (ii) une molécule reporteur liée à une microbille marquée différemment, les microbilles des différentes variétés de duplex ayant des marqueurs d'éléments métalliques différents,

et la détection simultanée et séparée des marqueurs des microbilles marquées différemment par analyse de la fluorescence X caractéristique de chaque élément métallique différent.

**17.** Procédé pour tester des séquences génétiques, qui comprend :

le mélange dans un milieu aqueux, dans des conditions de test prédéterminés, (I) d'un matériel génétique qui comprend une séquence qui doit être testée (séquence cible) et (II) de duplex nucléotidiques faiblement hybridés constitués par (a) une sonde génétique pour ladite séquence cible, ladite sonde génétique étant liée à une microbille marquée, et par (b) une molécule homopolymérique, la séquence cible comprenant une séquence sens caractéristique et la sonde génétique comprenant (1) une séquence anti-sens complémentaire de la séquence sens caractéristique de ladite séquence cible et (2) une séquence hybridable conçue pour s'hybrider avec ledit homopolymère, ladite sonde génétique étant capable de s'hybrider plus fortement avec ladite séquence cible qu'avec ledit homopolymère dans lesdites conditions de test prédéterminées;

de sorte que les séquences cibles s'hybrident avec les sondes génétiques des duplex sonde-homopolymère pour former des duplex cible-sonde et les microbilles marquées portées par les sondes génétiques des duplex cible-sonde formés sont dissociées desdits homopolymères;

la ségrégation des duplex sonde-homopolymère restants d'avec les duplex cible-sonde;

et la détection des marqueurs des microbilles marquées dans les duplex sonde-homopolymère restants pour mesurer la diminution du nombre de marqueurs dans les duplex sonde-homopolymère fournis initialement.

**18.** Procédé selon la revendication 17, dans lequel les homopolymères des duplex sonde-homopolymère sont liés à un substrat solide en un emplacement fixé.

**19.** Procédé selon la revendication 17 ou 18, dans lequel un certain nombre de nucléotides qui sont capables de former des paires de bases avec ledit homopolymère sont constitués par ladite séquence hybridable ou par ladite séquence hybridable et par la séquence anti-sens de la sonde génétique.

**20.** Procédé selon la revendication 19, dans lequel le nombre de nucléotides formateurs de paires de bases est suffisant dans les conditions de test prédéterminées pour que la sonde génétique et l'homopolymère dudit duplex sonde-homopolymère restent hybridés en l'absence desdites séquences cibles, et est insuffisant pour qu'ils restent hybridés dans les conditions réactionnelles lorsque des séquences cibles sont présentes dans le milieu aqueux, du fait du blocage de certains desdits nucléotides formateurs de paires de bases par ladite séquence cible.

**21.** Procédé selon la revendication 20, dans lequel les nucléotides formateurs de paires de bases sont constitués par la séquence hybridable et par la séquence anti-sens de la sonde génétique, et les nucléotides formateurs de paires de bases de ladite séquence anti-sens sont situés en position adjacente à ladite séquence hybridable.

**22.** Procédé selon l'une quelconque des revendications 17 à 21, dans lequel les conditions du test choisies parmi la température et le pH sont prédéterminées de manière que la sonde génétique forme un duplex faiblement hybridé avec l'homopolymère et un duplex fortement hybridé avec la séquence cible.

**23.** Procédé selon la revendication 22, dans lequel le test est réalisé à une température juste inférieure à la température de fusion du duplex de la sonde génétique et de l'homopolymère.

**24.** Procédé selon la revendication 23, dans lequel, après avoir mesuré tout d'abord une diminution du nombre de marqueurs des duplex sondehomopolymère, la température du test est abaissée à une température inférieure à la température de fusion d'un duplex qui peut être formé entre (a) les nucléotides formateurs de paires de bases constitués par la séquence hybridable de ladite sonde génétique et (b) ledit homopolymère, et les marqueurs des microbilles marquées dans les duplex sonde-homopolymère sont détectés pour mesurer une augmentation du nombre de marqueurs par rapport au nombre de la première mesure.

**25.** Procédé selon l'une quelconque des revendications 17 à 24, dans lequel lesdites microbilles sont marquées avec des composés colorés, des composés qui sont fluorescents en éclairement ultraviolet, des traceurs radioactifs ou avec des éléments ou composés métalliques détectables par fluorescence X.

**26.** Procédé selon la revendication 25, dans lequel les microbilles sont marquées avec des éléments ou composés métalliques et les microbilles marquées dans les duplex sonde-homopolymère sont détectées par analyse de fluorescence X.

**27.** Procédé selon la revendication 18, dans lequel les microbilles sont marquées avec des éléments ou composés métalliques et les microbilles marquées liées à des duplex sonde-homopolymère sont détectées par analyse de fluorescence X à l'aide d'une source de rayons X ou d'électrons pour irradier les duplex sonde-homopolymère recueillis sur ledit substrat solide et d'un détecteur de fluorescence X capable d'observer un angle solide étroit, et l'emplacement fixé dudit substrat solide est choisi en fonction dudit détecteur et dudit milieu aqueux de manière que ledit détecteur observe essentiellement les rayons X fluorescents émis par lesdits duplex sonde-homopolymère recueillis sans observer un nombre sensible de rayons X fluorescents émis par les duplex cible-sonde dans ledit milieu aqueux.

**28.** Procédé selon la revendication 26 ou 27, comprenant en outre le mélange dans le milieu aqueux, en tant que témoin, d'une quantité connue d'une séquence cible connue et de duplex nucléotidiques faiblement hybridés supplémentaires, lesdits duplex supplémentaires étant constitués par (c) une sonde génétique pour ladite séquence cible connue et par (d) une molécule homopolymérique, ladite sonde génétique (c) étant liée à une microbille marquée, ladite microbille étant marquée avec un élément métallique différent du marqueur de la sonde génétique (a) pour la cible inconnue, et ladite sonde génétique (c) étant capable de s'hybrider plus fortement avec ladite séquence cible connue qu'avec ledit homopolymère (d) dans lesdites conditions de test prédéterminées;

la détection simultanée est séparée (I) des marqueurs des microbilles marquées dans les duplex sonde (a)-homopolymère (b) restants et (II) des marqueurs des microbilles marquées dans les duplex sonde (c)-homopolymère (d) restants, par analyse de la fluorescence X caractéristique desdits éléments métalliques différents;

et l'étalonnage du test des séquences cibles par rapport au test du témoin.

29. Procédé selon la revendication 26, 27 ou 28, dans lequel ledit matériel génétique comprend différents types de séquences cibles qui doivent être testés, et lesdits types différents de séquences cibles sont testés par mélange dans le milieu aqueux de différentes variétés de duplex nucléotidiques faiblement hybridés, chaque variété différente de duplex étant constituée par (a) un type différent de sonde génétique, chaque type étant capable de s'hybrider avec un type particulier parmi lesdits types différents de séquences cibles qui doivent être testés et chaque type de sonde génétique étant lié à une microbille marquée différemment, et par (b) une molécule homopolymérique, les microbilles des variétés différentes de duplex ayant des marqueurs d'éléments métalliques différents;

et la détection simultanée et séparée des marqueurs des microbilles marquées différemment dans les duplex sonde-homopolymère restants par analyse de la fluorescence X caractéristique de chaque élément métallique différent.

30. Trousse pour la mise en oeuvre du procédé selon la revendication 1, pour tester des séquences génétiques dans un milieu aqueux contenant un matériel génétique qui comprend une séquence génétique cible qui doit être testée, ladite trousse comprenant un milieu aqueux contenant ensemble dans ledit milieu ou dans des parties séparées du milieu les composants suivants:

A. Des duplex nucléotidiques faiblement hybridés constitués par (i) une sonde génétique pour ladite séquence cible et par (ii) une molécule reporteur liée à une microbille marquée; et

B. Une multiplicité d'homopolymères comprenant des nucléotides capables de s'hybrider sélectivement avec les molécules reporteurs (ii) lorsque lesdites molécules reporteurs ne sont pas hybridées avec les sondes génétiques (i).

31. Trousse selon la revendication 30, dans laquelle ladite séquence cible, ladite sonde génétique, ladite molécule reporteur, ladite microbille marquée et lesdits homopolymères comprenant des nucléotides sont tels que définis dans l'une quelconque les revendications 2,7 à 9, ou 11 à 13.

32. Trousse selon la revendication 30 ou 31, comprenant en outre un substrat solide auquel les homopolymères sont liés.

33. Trousse selon la revendication 30, 31 ou 32, pour tester différents types de séquences cibles, dans laquelle le composant A. comprend en outre différentes variétés de duplex nucléotides hybridés, chaque variété différente de duplex étant constituée par (i) un type différent de sonde génétique, chaque type étant capable de s'hybrider avec un type particulier parmi lesdits types différents de séquences cibles qui doivent être testés, et par (ii) une molécule reporteur liée à une microbille marquée différemment, les microbilles des différentes variétés de duplex ayant des marqueurs d'éléments métalliques différents, lesdites variétés différentes de duplex étant contenues dis le même milieu aqueux ou dans des parties séparées de celui-ci.

34. Trousse selon l'une quelconque des revendications 30 à 33, comprenant en outre:

C. Une quantité connue d'une séquence cible connue en tant que témoin, contenue dans une partis du milieu aqueux qui est séparée du milieu aqueux contenant le composant A.; et

D. Des duplex nucléotidiques faiblement hybridés supplémentaires, lesdits duplex supplémentaires étant constitués par (iii) une sonde génétique pour ladite séquence cible connus et par (iv) une molécule reporteur liée à une microbille marquée, ladite microbille étant marquée avec un élément métallique différent du marqueur de la molécule reporteur (ii), ladite sonde génétique (iii) étant capable de s'hybrider plus fortement avec ladite séquence cible connue qu'avec ladite molécule reporteur (iv) dans des conditions de test prédéterminées.

35. Trousse pour la mise en oeuvre du procédé selon la revendication 17, pour tester des séquences génétiques dans un milieu aqueux contenant un matériel génétique qui comprend une séquence génétique cible qui doit être testée, ladite trousse comprenant un milieu aqueux contenant un composant (A) qui comprend des duplex nucléotidiques faiblement hybridés constitués par (a) une sonde génétique pour ladite séquence cible, ladite sonde génétique étant liée à une microbille marquée, et par (b) une molécule homopolymérique, ladite sonde génétique étant capable de s'hybrider plus fortement avec ladite séquence cible qu'avec ledit homopolymère dans des conditions de test prédéterminées.

36. Trousse selon la revendication 35, dans laquelle ladite séquence cible, ladite sonde génétique, ladite microbille marquée et ladite molécule homopolymérique sont telles que définies dans l'une quelconque des revendications 19 à 21, 25 ou 26.

37. Trousse selon la revendication 35 ou 36, comprenant en outre un substrat solide auquel les homopolymères sont liés.

38. Trousse selon la revendication 35, 36 ou 37, pour tester différents types de séquences cibles, dans laquelle le composant (A) comprend en outre différentes variétés de duplex nucléotidiques hybridés, chaque variété différente de duplex étant constituée par (a) un type différent de sonde génétique, chaque type étant capable de s'hybrider avec un type particulier parmi lesdits types différents de séquences cibles qui doivent être testés et chaque type de sonde génétique état lié à une microbille marquée différemment, et par (b) une molécule homopolymérique, les microbilles des variétés différentes de duplex ayant des marqueurs d'éléments métalliques différents, lesdites variétés différentes de duplex étant contenues dans le même milieu aqueux ou dans des parties séparées de celui-ci.

39. Trousse selon l'une quelconque des revendications 35 à 38, comprenant en outre:
B. Une quantité connue d'une séquence cible connue en tant que témoin, contenue dans une partie du milieu aqueux qui est séparée du milieu aqueux contenant le composant A.; et
C. Des duplex nucléotidiques faiblement hybridés supplémentaires, lesdits duplex supplémentaires étant constitués par (c) une sonde génétique pour ladite séquence cible connue et par (d) une molécule homopolymérique, ladite sonde génétique (c) étant liée à une microbille marquée, ladite microbille étant marquée avec un élément métallique différent du marqueur de la sonde génétique (a) pour la séquence cible inconnue, et ladite sonde génétique (c) étant capable de s'hybrider plus fortement avec ladite séquence cible connue qu'avec ledit homopolymère (d) dans des conditions de test prédéterminées.

A

B

C

FIG.1

FIG.2